# EUROPEAN PATENT APPLICATION

(11) **EP 1 797 850 A1**
(43) Date of publication of application: **20.06.2007**
(21) Application number: 06126082.4
(22) Date of filing: 14.12.2006
(51) Int. Cl.: A61F 13/00, A61L 15/18, A61L 15/42

(54) **Devices and methods for promoting the formation of blood clots at dialysis access sites**

(30) Priority: 16.12.2005 US 303607
(71) Applicant: Huey, Raymond J., Orange, CT 06477 (US); Horn, Jeffrey L., Rocky Hill, CT 06067 (US)
(72) Inventor: Huey, Raymond J., Orange, CT 06477 (US); Horn, Jeffrey L., Rocky Hill, CT 06067 (US)
(74) Representative: Berglund, Stefan

(57) **Abstract**

In a device for promoting the clotting of blood at a dialysis access site, a cuff (10) is provided and has an outer shell (12) and an inner lining (14). The cuff is constrictable around a body portion of a patient on which a wound associated with the dialysis access site is located. A molecular sieve material is attached to the cuff so that when treating the wound, at least a portion of the molecular sieve material contacts blood emanating from the wound.

## Description

### TECHNICAL FIELD

The present invention relates generally to blood clotting devices and, more particularly, to blood clotting materials, devices incorporating such materials, and methods for the delivery of such materials for use in controlling bleeding at dialysis access sites.

### BACKGROUND OF THE INVENTION

Blood is a liquid tissue that includes red cells, white cells, corpuscles, and platelets dispersed in a liquid phase. The liquid phase is plasma, which includes acids, lipids, solubilized electrolytes, and proteins. The proteins are suspended in the liquid phase and can be separated out of the liquid phase by any of a variety of methods such as filtration, centrifugation, electrophoresis, and immunochemical techniques. One particular protein suspended in the liquid phase is fibrinogen. When bleeding (minor discharges of blood) or hemorrhaging (discharges of copious amounts of blood) occurs, the fibrinogen reacts with water and thrombin (an enzyme) to form fibrin, which is insoluble in blood and polymerizes to form clots.

The kidneys are excretory organs that form part of the urinary system. In humans, one kidney is located on each side of the spine below the liver and the spleen in the posterior part of the abdomen and behind the peritoneum (the lining of the abdominal cavity). Both kidneys are "bean-shaped" and have a concave side that faces the medial direction (toward the spine). On the medial side of each kidney is an opening that admits the renal (kidney) artery, the renal vein, nerves and the ureter.

The kidneys filter wastes such as urea and other toxins from the bloodstream and excrete them as urine. This basic function of the kidneys is effectuated by nephrons, of which more than one million are located in each normal adult kidney. Nephrons regulate water and soluble matter (particularly electrolytes) in the body by filtering the blood, reabsorbing necessary components, and secreting the toxins and unnecessary components with urine.

When one or both kidneys fail to provide adequate renal functions or have failed to operate altogether, the functions of the kidneys may be performed using artificial means, viz., dialysis. Dialysis is a method of removing toxins from blood when the kidneys are unable to do so or when their capacity to do so is diminished. Dialysis therapy is most frequently used in instances in which the kidneys do not provide adequate renal function, but it may also be utilized in instances in which it is desirable to remove toxins in the form of drugs or poisons from the blood stream.

One type of dialysis is hemodialysis, which works by circulating the blood through filters having semi-permeable membranes incorporated therein. In hemodialysis, the filters through which the blood is circulated are external to the body of the patient. To externally circulate the blood, an arteriovenous fistula (an artificial connection of an artery and a vein) is surgically formed in the arm (or sometimes the leg) of the patient, and blood is taken from the fistula, dialyzed, and returned to the fistula. In a typical dialysis treatment, anticoagulant drugs such as Heparin are administered to the patient to provide less restrictive blood flow from the patient, through the dialysis apparatus, and back to the patient.

One known therapy that is used for the cessation of blood flow from the dialysis access site after a dialysis treatment is complete is the application of pressure. Generally, pressure is applied on the fistula to cause blood to clot at the openings through which the blood was removed and the dialyzed blood returned. Because of the use of anticoagulant drugs to facilitate the blood flow, the clotting function of the blood is hampered. Given that the dialysis process is a lengthy one, the slow clotting effect serves only to increase the length of time for an already unpleasant experience. Thus, the patient often experiences discomfort. Furthermore, because complications may arise due to the slow clotting effect, a dialysis patient is typically monitored by health care personnel until the blood flowing from the wounds made in the fistula sufficiently clots.

Accordingly, there is a need for an improved device and method of facilitating clotting that can quickly stop the bleeding associated with dialysis, which may in turn alleviate at least some of the discomfort experienced by the patient.

Based on the foregoing, it is a general object of the present invention to provide devices for controlling bleeding from dialysis access sites and methods of their use that overcome the problems with or improve upon the prior art.

### SUMMARY OF THE INVENTION

According to one aspect, the present invention resides in a device for promoting the clotting of blood at a dialysis access site. The device includes a cuff having an outer shell and an inner lining and a molecular sieve material attached to the cuff. The cuff is constrictable around a body portion of a patient on which a bleeding site associated with the dialysis access site is located. The molecular sieve material contacts blood emanating from the bleeding site to produce a clotting effect.

In other aspects, the present invention resides in more particular forms of the devices for clotting blood at dialysis access sites. One such device comprises a cuff having an outer shell and an inner lining; a strap attached to the outer shell and configured to constrict the cuff around a body portion on which a wound associated with the dialysis access site is located; a pad attached to the inner lining such that when a patient uses the device, the pad registers with the wound; and a molecular sieve material in the form of a zeolite attached to the pad.

Another device comprises a cuff having a bladder located between the outer shell and the inner lining; a pad attached to the inner lining; and the zeolite material attached to the pad. The bladder is inflatable to constrict the cuff around a body portion of the patient.

Yet another device comprises a cuff having an outer shell and an inner lining; at least one elastic member located on the cuff; a pad attached to the inner lining; and a zeolite material attached to the pad. The elastic member is configured to constrict the cuff around a body portion of a patient.

In any of the devices, when treating the bleeding site, at least a portion of the zeolite material in the pad contacts blood emanating from the bleeding site to cause a clotting effect.

In yet another aspect, the present invention resides in a method of clotting blood emanating from a dialysis access site. The method comprises the steps of providing a device having a constrictable member having a molecular sieve material attached thereto; applying the device to a body part of a patient on which the dialysis access site is located; positioning the molecular sieve material where the dialysis access site is bleeding; and constricting the device to cause the molecular sieve material to contact the bleeding tissue. By doing so, a clotting effect is realized and the blood flowing from the dialysis access site is stemmed.

In any embodiment of the present invention, the molecular sieve material found to be particularly effective in causing blood to clot while minimizing any exothermic effect is a zeolite. The zeolite is attached to, incorporated into, or impregnated into the material forming the pad. The zeolite contains less than about 75% by weight silicon oxide, and preferably less than about 65% by weight silicon oxide; less than about 50% by weight aluminum oxide, and preferably less than about 40% by weight of aluminum oxide; less than about 30% by weight sodium oxide, and preferably less than about 20% by weight of sodium oxide; less about 30% by weight of calcium oxide, and preferably less than about 20% by weight of calcium oxide. Preferably, zeolite powder is impregnated into the substrate which in the preferred embodiment is a paper. While the material has been described as being impregnated into the paper, the present invention is not limited in this regard as the zeolite can also be adhesively attached to the paper without departing from the broader aspects of the invention.

One advantage of the present invention is that upon use of any of the devices of the present invention to treat a bleeding wound at a dialysis access site, the flow of blood is quickly and efficiently stopped. More specifically, even though the patient is administered anticoagulating drugs, the zeolite (or other molecular sieve material) quickly stems the flow of blood. Thus, discomfort caused to the patient is minimized or eliminated altogether. Furthermore, the time required by health care personnel to monitor the patient is reduced, thereby saving costs.

Another advantage is that the proper dose of blood clotting agent can be readily applied to a wound and removed therefrom upon the stopping of blood flow. Particularly when the blood clotting agent is incorporated into a pad, the pad can be readily removed from a sterilized packaging, applied, and removed from the wound. Guesswork, estimation, or calculation of the amounts of blood clotting agent for application to a bleeding wound is eliminated since there is a definite amount of material associated with the device. Accordingly, no material is wasted. Furthermore, no material is left at the wound site.

Still another advantage is that the powder or particle form of the particular zeolite material utilized allows the material to react less exothermically. The porous nature of the material still allows liquid blood constituents to be wicked away to cause thickening of the blood, thereby facilitating the formation of clots. Because the initial moisture content of the zeolite is controlled in a preferred embodiment, a less aggressive drawing of moisture from the blood is realized, which thereby tempers the exothermic effects experienced at the wound site.

### BRIEF DESCRIPTION OF THE DRAWINGS

FIG. 1 is a perspective view of a cuff, of the present invention, incorporating a zeolite material.
FIG. 2 is a perspective view of the cuff of FIG. 1 having a removable pad of zeolite material.
FIG. 3 is a perspective cutaway view of an alternate embodiment of a cuff, of the present invention, incorporating a zeolite material.
FIG. 4 is a perspective view of another alternate embodiment of a cuff, of the present invention, incorporating a zeolite material.
FIG. 5 is a schematic representation of a substrate incorporating particles of a zeolite material.
FIG. 6 is a schematic representation of an alternate embodiment of a substrate incorporating particles of a zeolite material.
FIG. 7 is a schematic representation of another alternate embodiment of a substrate.
FIG. 8 is a side view of the substrate of FIG. 7 illustrating one means of retaining a molecular sieve particle in the substrate.
FIG. 9 is a perspective view of another embodiment of the present invention wherein an adhesive bandage incorporates a zeolite material.
FIG. 9a is an exploded view of the embodiment of FIG. 9.

### DETAILED DESCRIPTION OF THE PREFERRED EMBODIMENTS

Disclosed herein are devices and methods for delivering materials to bleed sites, particularly wound sites associated with fistulas, grafts, or shunts formed at dialysis access sites to facilitate a blood dialysis therapy, for the purposes of promoting the clotting of blood. The devices are generally cuffs having hemostatic (blood clotting) agents incorporated therein such that the blood clotting agents contact sites from which blood emanates. By maintaining such contact, the blood clotting agents absorb at least portions of the liquid phases of the blood, thereby promoting clotting. In any device, the blood clotting agent is preferably a particulate molecular sieve material that can be maintained in direct contact with blood emanating from a wound.

The molecular sieve material used in the present invention may be a synthetic polymer gel, cellulose material, porous silica gel, porous glass, alumina, hydroxyapatite, faujasite, calcium silicate, zirconia, zeolite, or the like. Exemplary synthetic polymers include, but are not limited to, stylene-divinylbenzene copolymer, cross-linked polyvinyl alcohol, cross-linked polyacrylate, cross-linked vinyl ether-maleic anhydride copolymer, cross-linked stylene-maleic anhydride copolymer or cross-linked polyamide, and combinations thereof.

The molecular sieve material is preferably a zeolite. As used herein, the term "zeolite" refers to a crystalline form of aluminosilicate having the ability to be dehydrated without experiencing significant changes in the crystalline structure. The zeolite may include one or more ionic species such as, for example, calcium and sodium moieties. Typically, the preferred zeolite is a powdered, friable material that is less than about 75% by weight silicon oxide, and preferably less than about 65% by weight silicon oxide; less than about 50% by weight aluminum oxide, and preferably less than about 40% by weight aluminum oxide; less than about 30% by weight sodium oxide, and preferably less than about 20% by weight of sodium oxide; less than about 30% by weight of calcium oxide, and preferably less than about 20% by weight of calcium oxide. The calcium portion contains crystals that are about 5 angstroms in size, and the sodium portion contains crystals that are about 4 angstroms in size. The preferred molecular structure of the zeolite is an "A-type" crystal, namely, one having a cubic crystalline structure that defines round or substantially round openings. The median size of the zeolite particle used is about 7 microns. However, the present invention is not limited in this regard as other particle sizes (as well as powder) are within the scope of the invention.

The zeolite may be mixed with or otherwise used in conjunction with other materials having the ability to experience dehydration functions without significant changes in crystalline structure. Such materials include, but are not limited to, magnesium sulfate, sodium metaphosphate, calcium chloride, dextrin, polysaccharides, combinations of the foregoing materials, and hydrates of the foregoing materials.

Various other materials may be mixed with, associated with, or incorporated into the zeolites to maintain an antiseptic environment at the wound site or to provide functions that are supplemental to the clotting functions of the zeolites. Exemplary materials that can be used include, but are not limited to, pharmaceutically-active compositions such as antibiotics, antifungal agents, antimicrobial agents, anti-inflammatory agents, analgesics (e.g., cimetidine, chloropheniramine maleate, diphenhydramine hydrochloride, and promethazine hydrochloride), bacteriostatics, compounds containing silver ions, and the like. Still other materials that can be incorporated to provide additional hemostatic functions include ascorbic acid, tranexamic acid, rutin, and thrombin. Botanical agents having desirable effects on the wound site may also be added.

Zeolites for use in the disclosed applications may be naturally occurring or synthetically produced. Numerous varieties of naturally occurring zeolites are found as deposits in sedimentary environments as well as in other places. Naturally occurring zeolites that may be applicable to the compositions described herein include, but are not limited to, analcite, chabazite, heulandite, natrolite, stilbite, and thomosonite. Synthetically produced zeolites that may also find use in the compositions and methods described herein are generally produced by processes in which rare earth oxides are substituted by silicates, alumina, or alumina in combination with alkali or alkaline earth metal oxides. One zeolite material found to be particularly useful in practicing the present invention is MOLSIV ADSORBENTS 5A, manufactured by UOP LLC of Des Plaines, Illinois. However, the present invention is not limited in this regard as other zeolite materials can be substituted without departing from the broader aspects of the present invention.

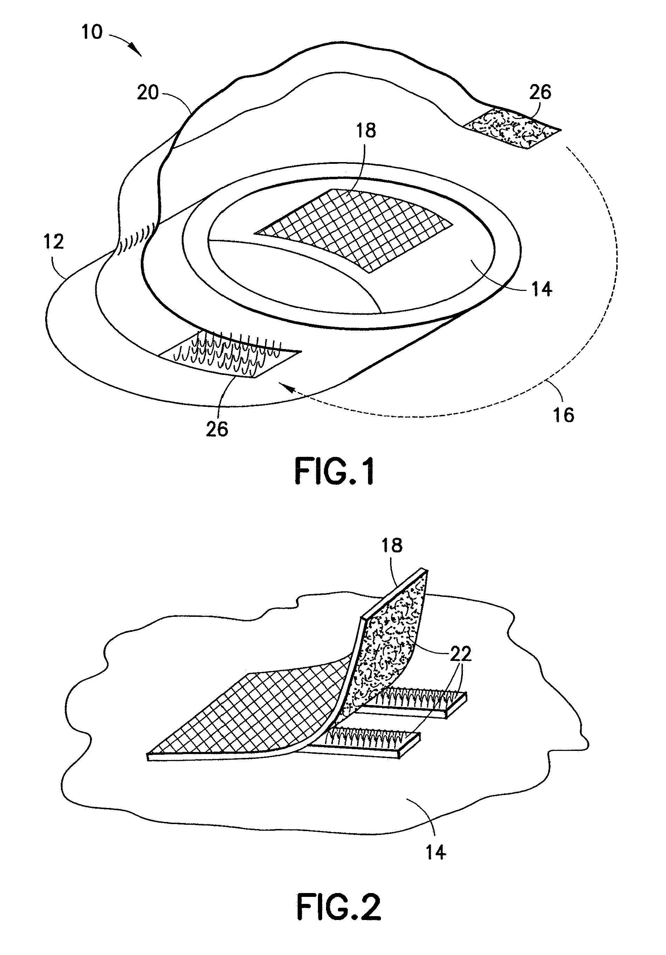

In one embodiment of the present invention shown in FIG. 1, one device that can be used by a patient to facilitate the clotting of blood at a dialysis access site is a cuff, which is shown at reference numeral 10 and is hereinafter referred to as "cuff 10." The cuff 10 is tubular in shape and comprises an outer shell 12 having an inner lining 14 attached thereto. A pad 18 incorporating a zeolite material is attached to at least a portion of the inner lining 14, the pad being located so as to allow the zeolite material to be maintained in contact with a wound inflicted as a result of a dialysis process. The pad 18 may be fixed to the inner lining 14 using stitching, adhesives, or the like. Alternately, the pad 18 may be removably attached to the inner lining 14 using any material that is suitable to allow the pad to be removed and a new pad reattached, as is shown in FIG. 2. One exemplary material that may be used to attach the pad 18 to the inner lining 14 is hook and loop material 22. Other materials that may be used include, but are not limited to, pressure sensitive adhesive, snaps, and the like.

The cuff 10 is constrictable around a body part (e.g., the arm or leg) of a patient. As shown in FIG. 1, at least one strap 20 is attached to the outer surface of the outer shell 12 to allow the cuff 10 to be tightened so as to allow the zeolite material of the pad 18 to be maintained against the bleed site with some amount of pressure. It is contemplated that the strap 20 is positionable around the outer shell 12 in the direction of an arrow 16 and fastenable using hook and loop material, as is shown at 26, although other means of fastening the strap (e.g., buttons, buckles, hooks and eyes, adjustable quick release clasps, and the like) are also within the scope of the invention.

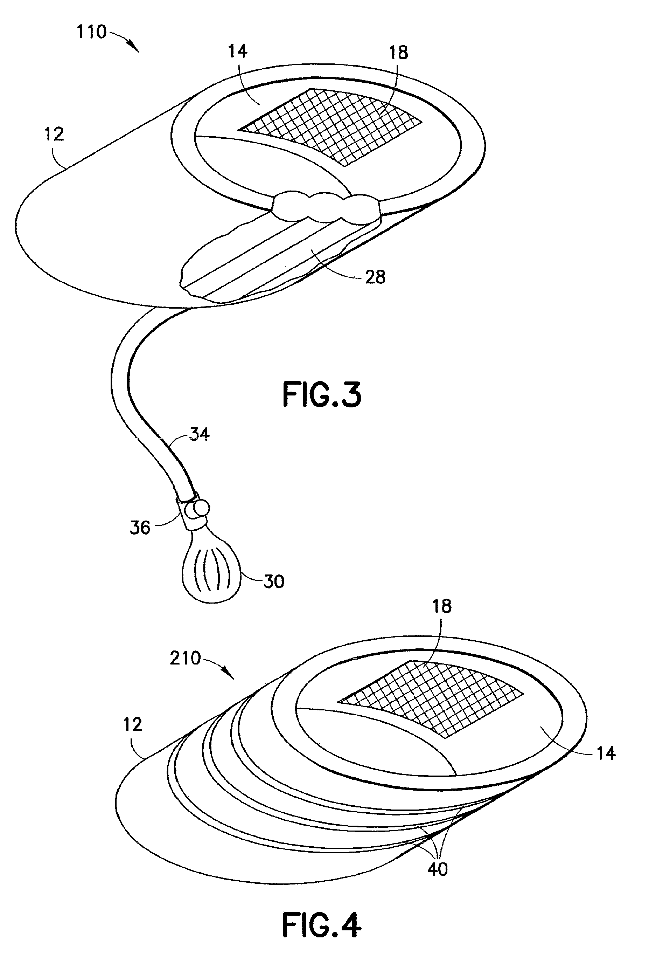

Referring now to FIG. 3, another embodiment of the cuff is shown generally at 110, in which the cuff again is tubular in shape and comprises the outer shell 12 having the inner lining 14 attached thereto, as well as the pad 18 attached to the inner lining. In cuff 110, a bladder 28 is positioned between the outer shell 12 and the inner lining 14. The bladder 28 allows the cuff 110 to be inflated to constrict about the body part of the patient, thereby applying pressure on the pad 18 and against the wound. The bladder 28 is inflatable using a bulb 30 that, when squeezed, fills the bladder with air through a hose 34 in a manner similar to that in which a blood pressure cuff is filled. The bulb 30 includes an exhaust valve 36 that can be manipulated to allow the cuff 110 to be readily deflated.

Referring now to FIG. 4, yet another embodiment of the cuff is shown generally at 210. Cuff 210 has the outer shell 12 and the inner lining 14. The outer shell 12 includes elastic members 40 attached along the peripheries of the openings at the ends of the tube, as is shown, to retain the cuff 210 on the arm or leg of the patient. At least one elastic member 40 may also be attached around the outer shell 12 at a point intermediate the ends of the tube to hold the pad 18 against the wound with some amount of pressure.

In any of the embodiments of the cuff described with reference to FIGS. 1-4, the cuff may be made to be disposable after a single use, or it may be sterilizable and reusable. In embodiments in which the cuff is inflatable (cuff 110, shown in FIG. 3) but also disposable, the bulb 30 and the hose 34 can be detached, sterilized, and reused on other cuffs. Also, with regard to the embodiments shown in FIGS. 3 and 4, the pad 18 may be removable from the inner lining 14 as is shown in FIG. 2.

Referring to FIGS. 1, 3, and 4, the outer shell 12 of the cuff is preferably nylon, rubber, plastic, or the like to facilitate the cleaning thereof. The inner lining 14 may be any suitable material that is compatible with and comfortable when placed against the skin of the patient. Preferably, the material of the inner lining 14 is non-allergenic and non-irritating. Exemplary materials for the inner lining 14 include, but are not limited to, cotton, paper, synthetic materials that are breathable (e.g., GORTEX), and the like.

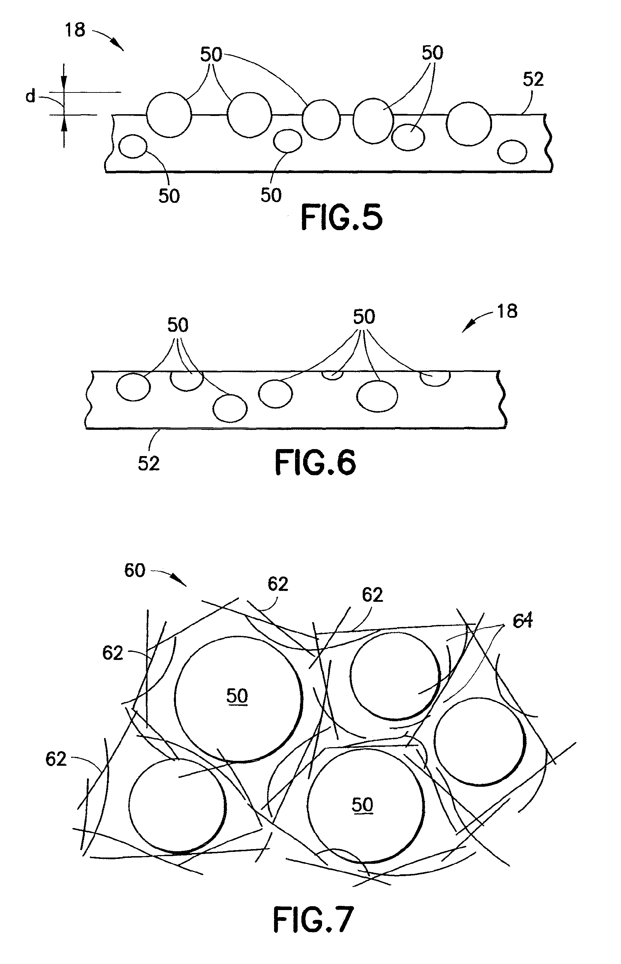

Referring now to FIGS. 5 and 6, exemplary embodiments of the pad 18 are shown. In the pad 18, the particles, which are shown at 50, may be embedded into a substrate 52. The particles 50 may be zeolite, as described above, or any other type of molecular sieve material. The substrate 52 may be any material that is suited to retain the zeolite particles 50. In FIG. 5, at least some of the particles 50 may protrude above an upper surface of the substrate 52 by a distance d. In FIG. 6, all of the particles 50 are shown as being at or below the surface of the substrate 52, with particles that would normally have been extending above the surface being cut, planed, shaved, crushed, or otherwise acted on to be level with the surface. In either embodiment, exemplary materials for the substrate 52 include, but are not limited to, cellulose-based materials (paper), polymers, open-cell foams, combinations of the foregoing, and the like.

Referring now to FIG. 7, another suitable substrate which may form the fabric of the pad (or be incorporated thereinto) is shown generally at 60. Substrate 60 is a porous web defined by interconnected, strands, filaments, strips of material, or fibers 62 such that the interconnection of the fibers retains the zeolite material in fine particulate form therein. The zeolite particles 50 can be incorporated into the porous web structure during formation of the substrate 60 or they can be impregnated into the finished substrate by conventional impregnation methods such as, but not limited to rolling. Various impregnation methods will produce differing loading levels for the zeolite materials and also differing degrees of bonding of the zeolite particles into the web.

The substrate 60 comprises the porous web and zeolite particles 50 retained thereon by impregnation into interstices 64 defined by the fiber of the web material. As illustrated, the substrate 60 is planar, and only a few zeolite particles 50 are shown for illustration purposes. While the zeolite particles 50 have been shown and described as being retained in interstices 64 defined by the fiber of the web material, the present invention is not limited in this regard, as the zeolite particles can be adhesively or otherwise bonded to the substrate without departing from the broader aspects of the present invention.

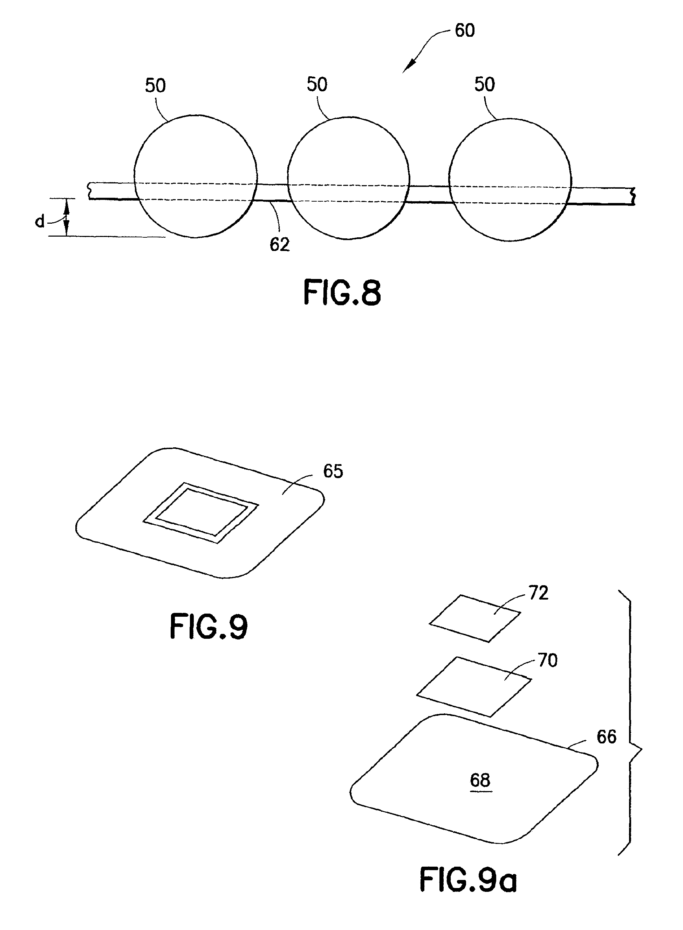

Referring now to FIG. 8, the interconnection of the fibers 62 defines the interstices 64 such that the zeolite particles 50 are retained but such that they extend out of the plane of the substrate 60 by a distance d. As such, the zeolite particles 50 are allowed to come into direct contact with flowing blood. Because the substrate 60 may partially surround the zeolite particles 50, a portion of the particles extend through the interstices. This allows the zeolite to directly contact tissue and thereby blood to which the blood clotting device is applied. Accordingly, blood emanating from the tissue contacts the zeolite particles 50, and the liquid phase thereof is wicked into the zeolite material, thereby facilitating the clotting of the blood. However, it is not a requirement of the present invention that the zeolite particles protrude out of the plane of the substrate.

With regard to the zeolite particles 50, however, less particle surface area is available for contact with blood as particle size increases. Therefore, the rate of clotting can be controlled by varying the particle size. Furthermore, the adsorption of moisture (which also has an effect on the exothermic effects of the zeolite) can also be controlled.

Referring to both FIGS. 7 and 8, the fiber that defines the porous web of the substrate 60 may be paper, polymer, cloth, or any suitable natural or synthetic material. Paper fibers can be any cellulose-based material (e.g., wood, cotton, and the like). One particular type of paper that is useful in practicing the present invention is surgical grade kraft paper. Cellulose derivatives such as cellulose esters (e.g., cellulose acetate), cellulose ethers (e.g., methylcellulose), and cellulose nitrates (e.g., nitrocellulose) are also within the scope of the cellulose-based materials described herein.

Non-woven natural and synthetic cloth substrates can also be employed. Such substrates allow the underlying skin or tissue to "breathe" thereby providing for longer contact with damaged tissue since gaseous exchange can still take place. Non-woven natural and synthetic cloth substrates include, but are not limited to, TYVEK, GORTEX, and the like.

Polymer substrates can be any suitable polymeric material drawn into fiber form. Solid matrices are also useful where the zeolite particles reside bound to the surface of a polymer sheet. Open-cell foam having porosity throughout the substrate to form a sponge structure is also desirable in some applications. The term "open-cell" as used herein shall be construed to mean that blood can pass into the cells to contact molecular sieve material resident inside the cells. Polyethylene solid or open cell sponge material can form such a substrate wherein the zeolite (or other molecular sieve particles) are bound in place but still allow intimate contact with the blood for desired clotting without leaving molecular sieve particles on the wound surface when the desired degree of clotting is achieved.

Synthetic polymeric plastics that are useful can be MYLAR (polyethylene terephthalate polyesters), polyethylene film, polypropylene film, polyethylene-polyamide laminated film, polyethylene-polyester laminated film, polypropylene-polyester laminated film, polyethylene-cellophane laminated film, and polyethylene-stretched polypropylene laminated film. Flexible, air permeable, high temperature resistant, bacteria-impermeable substrate material can preferably be made of non-woven polyester layers or polymeric fibrous materials such as polypropylene or polyester. The polyester can be located on either side of and bonded to a microporous membrane. Suitable polyesters include, but are not limited to REEMAY, which is available from BBA Fiberweb of Brentwood, Tennessee, and VERATEC. Hydrophobic fluoropolymers such as microporous polytetrafluoroethylene; polyvinylfluoride, polyvinylidenefluoride, polychlorotrifluoroethylene, polyfluoroethylenepropylene, perfluoroalkoxyethylene and tetrafluoroethylene (TFE) copolymers; chlorotrifluoroethylene and ethylene copolymers; and TFE and ethylene copolymers are also suitable. However, the present invention is not limited in this regard.

Natural and synthetic substrates can tolerate the dehydration temperatures used to assure that the zeolite material has the desired level of water present to control any exothermic reaction and the temperature associated therewith. The dehydration temperature can be as low as 200 degrees Centigrade. Higher temperatures up to 400 degrees Centigrade reduce the time required to dehydrate the zeolite. However, the present invention is not limited in this regard as other temperatures and dehydration methods known to those skilled in the pertinent art to which the present invention pertains can be employed without departing from the broader aspects of the present invention.

As shown in FIGs. 9 and 9a a bandage generally designated by the reference number 65 includes a sheet of material 66 having an adhesive applied to an attaching surface 68 thereof. An absorbent pad 70 is secured to the attaching surface 68. A second pad 72 is attached to the absorbent pad 70 and has molecular sieve material on an outwardly facing surface thereof. During use, the attaching surface is removably adhered to a user's skin surrounding a wound site. At least a portion of the molecular sieve material then comes into contact with blood emanating from the wound.

In any embodiment, the substrate 52, 60 can be fixed to the inner lining by any suitable means. Suitable means of attaching the substrate 52, 60 to the inner lining include, but are not limited to, welding, adhesive bonding, brazing, stitching, and the like.

In any embodiment, the control of the moisture content of the zeolite in the substrate is related to its effectiveness. The preferred moisture content is between about 5 and about 25% by weight, more preferably between about 7 and about 19% by weight, and most preferably between about 10 and about 15% by weight. The moisture content of the zeolite can be adjusted by drying and then re-hydrating, or a combination of drying and re-hydrating, such that the zeolite has the desired specific moisture content. Alternatively, the composition may be fully saturated with water and subsequently dried to a specific water content. In the drying of the zeolite, the bound water is removed to allow the crystalline structure of the zeolite to remain intact. In the re-hydration of the zeolite, the most active adsorption sites are hydrated first and then less active sites are hydrated. As the degree of hydration of the zeolite increases, the heat of hydration decreases. More specifically, when the composition is applied to the blood, water in the blood is adsorbed by the zeolite. Upon adsorption of this water, heat is generated. At higher levels of hydration (hydration of the zeolite prior to its application to blood), less heat is generated when the composition is applied to blood. Thus, when the composition is applied to blood directly at a wound site, the amount of heat transferred to the tissue surrounding the wound site is reduced.

The devices and methods of the present invention are particularly useful in therapies where it is desired to quickly and efficiently stop bleeding at dialysis access sites after the completion of a dialysis treatment. The present invention may further have applications in other therapies and surgical procedures in which the stopping of blood flow can be problematic, or in the treatment of wounds on the extremities. Such therapies, surgical procedures, and wound treatments may be for veterinary purposes.

Although this invention has been shown and described with respect to the detailed embodiments thereof, it will be understood by those of skill in the art that various changes may be made and equivalents may be substituted for elements thereof without departing from the scope of the invention. In addition, modifications may be made to adapt a particular situation or material to the teachings of the invention without departing from the essential scope thereof. Therefore, it is intended that the invention not be limited to the particular embodiments disclosed in the above detailed description, but that the invention will include all embodiments falling within the scope of the appended claims.

## Claims

1. A device for promoting the clotting of blood at a dialysis access site, said device **characterized by**:
a cuff (10) having an outer shell (12) and an inner lining (14), said cuff being constrictable around a body portion of a patient on which a wound associated with said dialysis access site is located; and
a molecular sieve material attached to said cuff;
wherein when treating said wound, at least a portion of said molecular sieve material is adapted to contacts blood emanating from said wound.

2. The device of claim 1, wherein said molecular sieve material is a zeolite.

3. The device of claim 2, wherein said zeolite comprises,
less than about 75% by weight of silicon oxide,
less than about 50% by weight of aluminum oxide,
less than about 30% by weight of sodium oxide, and
less than about 30% by weight of calcium oxide.

4. The device of any one of claims 1 - 3, wherein said molecular sieve material is disposed on a substrate to form a pad (18), said pad being removably attached to said inner lining (14).

5. The device of claim 4, wherein said pad (18) is attached to said inner lining (14) such that upon a patient using said device said paid registers with said wound.

6. The device of any one of claims 1 to 5, wherein said cuff (10) is constrictable using a strap (20) connected to said outer shell (12).

7. The device of claim 6, wherein said strap (20) is connectable back to said outer shell (12) using hook and loop material.

8. The device according to claim 6, wherein said strap (20) is attached to said outer shell (12) at a first point and being connectable to at least one of said outer shell and said strap at a second point to constrict said cuff around a body portion of a patient on which said wound is located.

9. The device of any one of claims 1 - 8, wherein said cuff (10) is constrictable using an inflatable bladder (28) positioned between said outer shell and said inner lining.

10. The device of claim 9, further comprising a squeezable bulb (30) for inflating said inflatable bladder (28).

11. The device of any one of claims 9 and 10, further comprising an exhaust valve (36) for deflating said inflatable bladder (28).

12. The device of any one of claims 1 - 11, wherein said cuff (10) is constrictable using an elastic member (40) attached along the peripheries of openings of said cuff.

13. The device of any one of claims 1 - 12, wherein said molecular sieve material is disposed on a substrate (52, 60) that is attached to said inner lining.

14. The device of claim 13, wherein a material from which said substrate is fabricated consists of cellulose-based materials, paper, polymers, cloths, foams, and combinations of the foregoing.

15. The device of any one of claims 13 and 14, wherein said substrate comprises a material selected from the group of materials consisting of non-woven natural cloth and non-woven synthetic cloth.

16. The device of any one of claims 13 - 15, wherein said substrate comprises a material selected from the group of materials consisting of polyesters, polyethylene terephthalate polyesters, polyethylene film, polypropylene film, polyethylene-polyamide laminated film, polyethylene-polyester laminated film, polypropylene-polyester laminated film, polyethylene-cellophane laminated film, polyethylene-stretched polypropylene laminated film, polytetrafluoroethylene, polyvinylfluoride, polyvinylidenefluoride, polychlorotrifluoroethylene, polyfluoroethylenepropylene, perfluoroalkoxyethylene, tetrafluoroethylene copolymers, chlorotrifluoroethylene and ethylene copolymers, tetrafluoroethylene and ethylene copolymers, and combinations of the foregoing.

17. The device of any one of claims 13 - 16, wherein said substrate (52, 60) is a porous web in which particles (50) of said molecular sieve material are retained.

18. The device of claim 17, wherein said porous web is defined by an interconnection of fibers (62) that define interstices in which said particles (50) of said molecular sieve material are retained.

19. The device of any one of claims 17 and 18, wherein said particles (50) of said molecular sieve material extend out of a plane of said substrate (52, 60).

20. A method of clotting blood emanating from a dialysis access site, said method **characterized by** the steps of:
providing a device having a constrictable member having a molecular sieve material attached thereto;
applying said device to a body part of a patient on which said dialysis access site is located;
positioning said molecular sieve material at a bleed site of said dialysis access site; and
constricting said device to cause said molecular sieve material to contact tissue of said bleed site.

21. The method of claim 20, wherein said step of constricting said device comprises holding said constrictable member tight with a strap.

22. The method of claim 20, wherein said step of constricting said device comprises inflating a bladder of said device.

23. The method of claim 20, wherein said step of constricting said device comprises holding said constrictable member tight with an elastic member.

24. The method of any one of claims 20 - 24, wherein said molecular sieve material is a zeolite.

25. A device for promoting the clotting of blood at a dialysis access site, said device having:
a sheet of material having an adhesive applied to an attaching surface thereof;
an absorbent pad coupled to said attaching surface; further **characterized by**
a second pad having molecular sieve material adhered thereto coupled to said absorbent pad; and wherein
said attaching surface is removably adhered to a user's skin surrounding a wound so that at least a portion of said molecular sieve material contacts blood emanating from said wound.
